# EUROPEAN PATENT SPECIFICATION

(11) **EP 0 266 659 B1**
(45) Date of publication and mention of the grant of the patent: **27.04.1994**
(21) Application number: 87115745.9
(22) Date of filing: 27.10.1987
(51) Int. Cl.: C07C 271/08

(54) **Process for the preparation of tertiary aralkyl monourethanes**
Verfahren zur Herstellung von tertiären Aralkylmonourethanen
Procédé de préparation de monouréthanes d'aralcoyle tertiaire

(30) Priority: 03.11.1986 US 926055
(43) Date of publication of application: 11.05.1988
(73) Proprietor: AMERICAN CYANAMID COMPANY, Wayne, NJ 07470-8426 (US)
(72) Inventor: Alexanian, Vazken Arsen, Darien Connecticut (US)
(74) Representative: Wächtershäuser, Günter, Prof. Dr.

(56) References cited:
- EP-A- 0 101 832

## Description

The present invention relates to a novel and improved process for the selective preparation of aralkyl monourethanes in high yield and purity from the acid-catalyzed addition reaction of divinyl aromatic hydrocarbons and carbamic acid esters. More particularly, it relates to the preparation of aralkyl monourethanes, such as meta- and para-isopropenyl-alpha, alpha-dimethylbenzyl urethane (TMU) from the addition of methyl carbamate and diisopropenyl benzenes (DIPEB). The aralkyl monourethanes are important as intermediates in the manufacture by thermal cracking of corresponding monoisocyanates, and these are useful as intermediates in the manufacture of crosslinking agents in resins and in other plastic applications.

It is generally known that selective additions of for example, carbamate esters to diolefinic compounds are difficult to carry out, and rarely provide the mono-product in good yield. In many cases, diolefins polymerize to give complex mixtures or predominantly the bis-adduct, e.g., the diurethane product.

Mueller and Merten, Chem. Ber., 98, 1091-1110 (1965) carry out the alkylation of urethane, i.e., carbamic acid ethyl ester with a number of cyclic and noncyclic olefins in the presence of acid catalyst to form N-substituted urethanes. For example, in Example VIII, Table 1 of this publication, the mono-olefin, vinylbenzene, is reported to be reacted with the ethyl ester of carbamic acid to produce the monourethane adduct in a yield of 47%.

In EP-A-0101 832 and in U.S. 4,439,616, Singh et al., a process is disclosed for preparing urethanes by the acid-catalyzed addition reaction of carbamic acid esters. In Col. 3, lines 38 through 44, this patent suggests that "the addition reaction can also be utilized with diolefins, such as a diisopropenylbenzene (DIPEB) to favor the production of a monourethane, such as isopropenyl-alpha, alpha-dimethylbensyl urethane (TMU)..." In none of their examples, however, do Singh et al. provide for high yields of the monourethane product relative to the bis-adduct. Instead, the selective mono-addition to DIPEB is observed principally by patent '616 as a by-product obtained in low yield, that can be recycled with additional carbamate to produce the diurethane. For example, a reaction at 45°C for 22 hours provides a mixture of 67% of the diurethane and 20% of the monourethane, which corresponds to a ratio of 0.3:1 of the latter to the former (Col. 4, lines 2-17).

It has now been surprisingly discovered that monourethanes, such as isopropenyl-alpha,alpha-dimethylbenzyl urethane (TMU) can be prepared in excellent yield and high purity by the selective acid-catalyzed addition reaction of a carbamic acid ester and a divinyl aromatic hydrocarbon, such as diisopropenyl benzene (DIPEB).

In accordance with the present invention, there is provided an improved process for preparation of a monourethane selected from the formula: 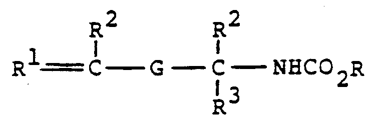
wherein R is alkyl of from 1 to 18 carbon atoms, R¹ is an alkylidene group having from 1 to 3 carbon atoms; and R² and R³ each are an alkyl group having from 1 to 3 carbon atoms, and G is a divalent aromatic hydrocarbon group, the improvement comprising selectively reacting
(a) a divinyl aromatic hydrocarbon of the formula 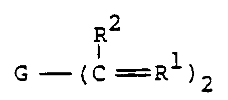 wherein R¹ and R² and G are as defined above, with
(b) a carbamic acid ester of the formula

   NH₂CO₂R

   wherein R is as defined above, in the presence of
(c) an effective catalytic amount of an acid at a temperature of from 25°C to less than 40°C until formation of a mixture of said monourethane compound and the corresponding diurethane compound in which said monourethane compound comprises a major proportion of 3.8 to 1 or greater is substantially complete.

Preferably, the monourethanes produced by the process are of the formula 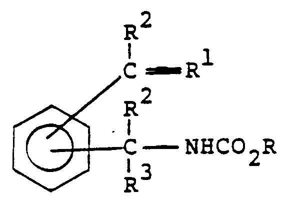
wherein R, R² and R³ are methyl and R¹ is methylene. In preferred embodiments the proportion of said monourethane to any corresponding diurethane is greater than 4 to 1, preferably 10:1 or greater.

The divinyl aromatic hydrocarbons useful as starting materials in the present invention are typically represented by the formula: 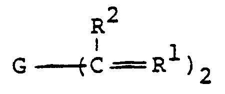
wherein R¹, R² and G are as defined above. These can comprise the compounds shown in Alexanian et al., U.S. 4,379,767. The G group can comprise phenyl, biphenyl or naphthyl, or such an aromatic hydrocarbon group having substituents such as halogen atoms, methyl or methoxy groups.

A family of preferred divinyl aromatic compounds used as starting materials comprise those of the formula: 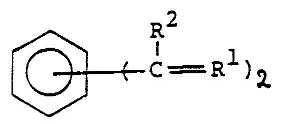
wherein R¹ and R² are as defined above, but especially preferably comprise methylene and methyl, respectively.

Other suitable compounds in this regard are divinylbenzene, divinylnaphthalene, para-diisopropenylbenzene (p-DIPEB), meta-diisopropenylbenzene (M-DIPEB), 3-vinyl-1-(1'-n-propyl) styrene, and 1-vinyl-3-isopropenylbenzene. Especially preferred are the para and meta isomers of diisopropenylbenzene.

The carbamic esters used as component (b) herein are compounds represented by the general formula: 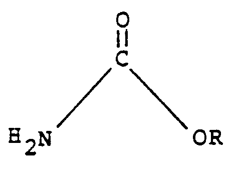
wherein R is as defined above, i.e., alkyl of from 1 to 18 carbon atoms, straight chain or branched. Examples of suitable alkyl carbamates are methylcarbamate, ethylcarbamate, propylcarbamate, butylcarbamate, octadecyl carbamate, 2-ethylhexylcarbamate or triacontyl carbamate. Especially preferred for use herein is the compound methyl-carbamate also known as carbamic acid methyl ester.

Methyl carbamate or other carbamates can be added in stoichiometric proportions to the divinyl aromatic hydrocarbon (a), but preferably the carbamate is in excess of stoichiometric in the range of from two moles to ten moles per mole of vinyl aromatic hydrocarbon (a). It is preferred in accordance with this invention to use from 200% to 600% stoichiometric excess of the carbamate, preferably about 400% excess of the carbamate.

The Lewis acid catalysts useful in this invention include soluble acid catalysts such as sulfuric acid, boron trifluoride etherate BF₃ . Et₂O and solid acid catalysts, such as sulfonic acid, e.g., Amberlyst-15® available from the Chemical Dynamics Corporation and polysulfonic acid resin, e.g., Nafion-H® available from the E.I. duPont De Nemours and Company. Other suitable acid catalysts include toluene sulfonic acid, dodecylbenzene sulfonic acid, hydrocarbon sulfate esters or hydrochloric acid. Special mention is made of sulfuric acid.

The amount of catalyst required to promote the addition of diolefin and carbamic acid ester is not critical and can be varied widely. Where substantial excess of carbamic acid ester is utilized the amount of catalyst, based on the vinyl aromatic hydrocarbon is typically 0.01 to 10 mole % and preferably 0.2 to 5 mole %, and especially preferably 1 mole %.

The reaction can take place in the absence of solvent or in the presence of solvents, such as methylene chloride, toluene, xylene or chlorobenzene.

Preferably the carbamate is heated to melting or dissolved. The catalyst is mixed into the carbamate or solution thereof, and the unsaturated hydrocarbon is then slowly added. When the reaction is complete the mixture is treated to remove or neutralize the catalyst. Unreacted carbamate ester is then separated by distillation in partial vacuum or by adding a large excess of water and filtering to separate insoluble urethane products from watersoluble carbamate ester.

If excess of carbamate is employed this can also be distilled off at partial vacuum and recovered. The recovered carbamate can be recycled along with catalyst. The reaction mixture of urethanes, unreacted carbamate ester, catalyst, and byproducts can also be separated by adding a large excess of an aqueous medium, for example, sodium carbonate solution to separate the urethane products as insolubles and also to neutralize the catalyst.

As will be hereinafter exemplified in the following section, it is advisable to monitor the formation of the monourethane product relative to bis-urethane formation. This can be accomplished by using gas liquid chromatography (GLC) area percent. Generally, when the ratio of monourethane to diurethane is approximately 4, as measured by GLC area percent, the pH of the reaction mixture can be adjusted to about 10 with base, e.g., sodium hydroxide, to stop the reaction. Reaction times will vary, but longer times, e.g., up to about 36 hours, are used at the lower temperatures and shorter times, e.g., from about 1 to about 12 hours, are used at the higher temperatures. Reaction times can be optimized by periodically measuring the ratios of products, e.g., by GLC, as mentioned above. The organic solvent can be removed using conventional methods, e.g., by drying with magnesium sulfate and evaporating in vacuo, leaving a residual oil which can be distilled to give unreacted vinyl aromatic hydrocarbon (a), unreacted carbamic acid ester (b), the desired monourethane with a substantially smaller quantity of the diurethane and a fraction comprising a complex mixture of components, typically by-products of the reaction. The residue can be further refined by dissolving in an organic solvent, e.g., methylene chloride and then filtering. The addition of a high boiling organic solvent, such as hexane, will separate out from the residue the diurethane.

The following examples illustrate the novel process of the present invention. They are not to be construed to limit the claims in any manner whatsoever.

### EXAMPLE 1

A solution of m-diisopropenylbenzene (79.0 g, 0.500 mol) in methylene chloride (125 ml) was added to a mixture of methyl carbamate (150 g, 2.00 mol) and sulfuric acid (0.49 g, 0.005 mol, 1 mole %). The resulting mixture was allowed to react for 16 hours (overnight) at 40°C. When the ratio of the monourethane to bis-urethane reached 4.2, as determined by GLC area percent, the pH of the reaction mixture was adjusted to 10 with the addition of a 2N sodium hydroxide solution to stop the reaction. The unreacted methyl carbamate was removed by washing with water (6 x 150 ml). The methylene chloride solution was dried with (MgSO₄), and then evaporated in vacuo at 60°C. The residual oil was distilled using a 3 inch Vigreaux column to give methyl carbamate (0.52 g, bp 30-57°C/1.33 mbar(1 mm Hg)), m-diisopropenyl benzene (39.2 g, 49.6%, bp 57-80°C/1.33 mbar(1 mm Hg)), a fraction (1.3 g) consisting of a complex mixture (bp 80-110°C/1,33 mbar(1 mm Hg)), and methyl N-(3-isopropenyl-alpha, alpha-dimethylbenzyl) carbamate (23.5 g, 34% based on olefin consumed, bp 110-120°C/1 mm Hg). The residue was then dissolved in methylene chloride (20 ml) and filtered. Addition of hexane gave the dimethyl N,N,-(alpha,alpha,alpha,alpha-tetramethyl-m-xylylene)dicarbamate (6.75 g, 9% based on olefin consumed, mp 128-129°C). The ratio of isolated monourethane to diurethane was 3.8.

### EXAMPLE 2

The procedure of Example 1 was repeated with the exception that the reaction was stopped at 2 hours/minutes instead of overnight. Analysis of the unreacted m-diisopropenyl by GLC indicated that only about 10% had been converted in the reaction. The ratio of the monourethane to diurethane (TMU/TMXDU) was 23:1 with about 2% DIPEB unaccounted for, as determined by GLC analysis.

The results in Example 2 indicate that higher ratios of the monourethane, TMU, relative to the bis-urethane, TMXDU, can be obtained at lower conversions of the reactant, DIPEB.

The above-mentioned publication and patent are incorporated herein by reference.

Many variations will suggest themselves to those skilled in the art. For example, instead of meta-diisopropenylbenzene, other vinyl aromatic hydrocarbons, such as para-diisopropenylbenzene and 1-vinyl-3-isopropenylbenzene can be used. For example, instead of using the methyl ester of carbamic acid in the addition reaction, the ethyl, propyl, butyl or any of the higher alkyl esters of carbamic acid can be used. For example, instead of using sulfuric acid, boron trifluoride etherate and phosphoric acid can be used as acid catalysts.

## Claims

1. In a process for the preparation of a monourethane selected from the formula: 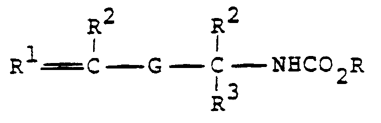 wherein R is alkyl of from 1 to 18 carbon atoms, R¹ is an alkylidene group having from 1 to 3 carbon atoms; and R² and R³ each are an alkyl group having from 1 to 3 carbon atoms, and G is a divalent aromatic hydrocarbon group, the improvement comprising selectively reacting
(a) a divinyl aromatic hydrocarbon of the formula 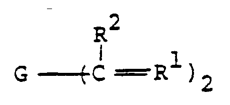 wherein R¹ and R² and G are as defined above, with
(b) a carbamic acid ester of the formula
NH₂CO₂R
wherein R is as defined above, in the presence of
(c) an effective catalytic amount of an acid at a temperature of from 25°C to less than 40°C until formation of a mixture of said monourethane compound and the corresponding diurethane compound in which said monourethane compound comprises a major proportion of 3.8:1 or greater is substantially complete.

2. A process as defined in Claim 1 wherein said monourethane is of the formula: 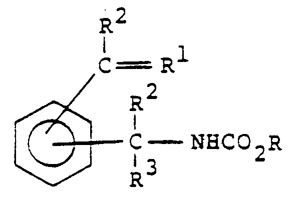 wherein R, R² and R³ are methyl R¹ is methylene and the proportion of said monourethane to any corresponding diurethane is 4.0:1 or greater.

3. A process as defined in Claim 1 in which from 2 to 10 moles of carbamic acid ester (b) are reacted per mole of divinyl aromatic hydrocarbon (a).

4. A process as defined in Claim 1 in which 4 moles of carbamic acid (b) are reacted per mole of divinyl aromatic hydrocarbon (a).

5. A process as defined in Claim 1 wherein said divinyl aromatic hydrocarbon (a) is selected from paradiisopropenylbenzene, meta-diisopropenylbenzene, 1-vinyl3(1'-n-propyl) styrene, and 1-vinyl-3-isopropenylbenzene.

6. A process as defined in Claim 1 wherein said divinyl aromatic hydrocarbon (a) is para-diisopropenylbenzene.

7. A process as defined in Claim 1 wherein said divinyl aromatic hydrocarbon (a) is meta-diisopropenylbenzene.

8. A process as defined in Claim 1 wherein said acid (c) is selected from sulfuric acid, boron trifluoride etherate, phosphoric acid, hydrogen chloride, sulfonic acid resins, hydrogen fluoride, alkyl sulfonic acid, sulfate esters and Lewis acid.

9. A process as defined in Claim 1 wherein said acid (c) is sulfuric acid.

10. A process as defined in Claim 1 including the step of recovering said monourethane compound substantially free of any diurethane compound and any unreacted divinyl aromatic hydrocarbon.

## Patentansprüche

1. Verfahren zum Herstellen eines Monourethans ausgewählt aus der Formel: 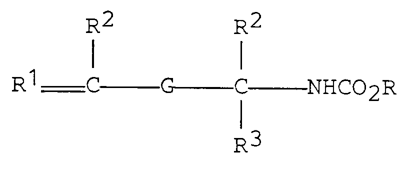 worin R Alkyl mit 1 bis 18 Kohlenstoffatomen, R¹ eine Alkylidengruppe mit 1 bis 3 Kohlenstoffatomen ist und R² und R³ jeweils eine Alkylgruppe mit 1 bis 3 Kohlenstoffatomen sind und G eine zweiwertige aromatische Kohlenwasserstoffgruppe ist, wobei die Verbesserung die selektive Umsetzung
(a) eines aromatischen Divinvlkohlenwasserstoffes der Formel 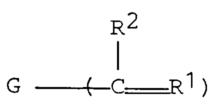 worin R¹ und R² und G die oben genannte Bedeutung haben, mit
(b) einem Carbaminsäureester der Formel
NH₂CO₂R
worin R die oben genannte Bedeutung hat, in Gegenwart
(c) einer wirksamen katalytischen Menge einer Säure bei einer Temperatur von 25°C bis weniger als 40°C umfaßt, bis die Bildung einer Mischung der Monourethan-Verbindung und der entsprechenden Diurethan-Verbindung, in der die Monourethan-Verbindung einen Hauptanteil von 3,8:1 oder mehr umfaßt, im wesentlichen abgeschlossen ist.

2. Verfahren nach Anspruch 1, worin das Monourethan die Formel hat: 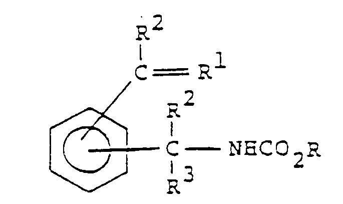 worin R, R² und R³ Methyl sind, R¹ Methylen ist und der Anteil des Monourethans zu einem entsprechenden Diurethan 4,0:1 oder mehr beträgt.

3. Verfahren nach Anspruch 1, bei dem von 2 bis 10 Mole Carbaminsäureester (b) pro Mol des aromatischen Divinylkohlenwasserstoffes (a) umgesetzt werden.

4. Verfahren nach Anspruch 1, bei dem 4 Mole Carbaminsäureester (b) pro Mol des aromatischen Divinvlkohlenwasserstoffes (a) umgesetzt werden.

5. Verfahren nach Anspruch 1, worin der aromatische Divinylkohlenwasserstoff (a) ausgewählt ist aus p-Diisopropenylbenzol, m-Diisopropenylbenzol, 1-Vinyl-3(1'-n-propyl)styrol und 1-Vinyl-3-isopropenylbenzol.

6. Verfahren nach Anspruch 1, worin der aromatische Divinylkohlenwasserstoff (a) p-Diisopropenylbenzol ist.

7. Verfahren nach Anspruch 1, worin der aromatische Divinylkohlenwasserstoff (a) m-Diisopropenylbenzol ist.

8. Verfahren nach Anspruch 1, worin die Säure (c) ausgewählt ist aus Schwefelsäure, Bortrifluorid-etherat, Phosphorsäure, Chlorwasserstoff, Sulfonsäureharzen, Fluorwasserstoff, Alkylsulfonsäure, Sulfatestern und Lewissäure.

9. Verfahren nach Anspruch 1, worin die Säure (c) Schwefelsäure ist.

10. Verfahren nach Anspruch 1, das die Stufe der Gewinnung der Monourethan-Verbindung im wesentlichen frei von irgendeiner Diurethan-Verbindung und irgendwelchem unumgesetzten aromatischen Divinylkohlenwasserstoff einschließt.

## Revendications

1. Dans un procédé de préparation d'un mono-uréthane choisi dans la formule : 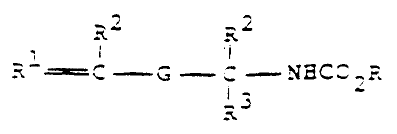 dans laquelle R est un groupe alkyle de 1 à 18 atomes de carbone, R¹ est un groupe alkylidène de 1 à 3 atomes de carbone et R² et R³ sont chacun un groupe alkyle comportant de 1 à 3 atomes de carbone, et G est un groupe hydrocarboné aromatique divalent, l'amélioration comprenant la réaction sélective
(a) d'un hydrocarbure divinyl-aromatique de formule 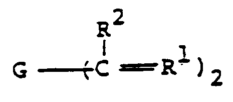 dans laquelle R¹ et R² et G sont tels que définis ci-dessus, avec
(b) un ester d'acide carbamique de formule
NH₂CO₂R
dans laquelle R est tel que défini ci-dessus, en présence
(c) d'une quantité efficace en tant que catalyseur d'un acide, à une température de 25°C à moins de 40°C, jusqu'à ce que la formation d'un mélange dudit composé mono-uréthane et du composé di-urethane correspondant, dans un rapport 3,8 : 1 ou plus dans lequel le composé mono-uréthane est majoritaire, soit essentiellement complète.

2. Procédé selon la revendication 1, dans lequel ledit mono-uréthane a pour formule : 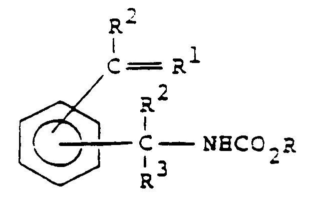 dans laquelle R, R² et R³ sont des groupes méthyle et R¹ est un groupe méthylène, et la proportion dudit mono-uréthane par rapport à tout di-uréthane correspondant est d'au moins 4,0 : 1.

3. Procédé selon la revendication 1, dans lequel on fait réagir de 2 à 10 moles d'ester d'acide carbamique (b) par mole d'hydrocarbure divinyl-aromatique (a).

4. Procédé selon la revendication 1, dans lequel on fait réagir 4 moles d'ester d'acide carbamique (b) par mole d'hydrocarbure divinyl-aromatique (a).

5. Procédé selon la revendication 1, dans lequel ledit hydrocarbure divinyl-aromatique (a) est choisi parmi le para-di-isopropénylbenzène, le méta-di-isopropénylbenzène, le 1-vinyl-3-(1'-n-propyl)styrène et le 1-vinyl-3-isupropénylbenzène.

6. Procédé selon la revendication 1, dans lequel ledit hydrocarbure divinyl-aromatique (a) est le para-di-isopropénylbenzène.

7. Procédé selon la revendication 1, dans lequel ledit hydrocarbure divinyl-aromatique (a) est le méta-di-isopropénylbenzène.

8. Procédé selon la revendication 1, dans lequel ledit acide (c) est choisi parmi l'acide sulfurique, le trifluorure de bore éthéré, l'acide phosphorique, le chlorure d'hydrogène, les résines d'acide sulfonique, le fluorure d'hydrogène, un acide alkylsulfonique, les esters sulfates et un acide de Lewis.

9. Procédé selon la revendication 1, dans lequel ledit acide (c) est l'acide sulfurique.

10. Procédé selon la revendication 1, comprenant l'étape de récupération dudit composé mono-uréthane essentiellement débarrassé de composé di-uréthane et d'hydrocarbure divinylaromatique n'ayant pas réagi.
